# EUROPEAN PATENT SPECIFICATION

(11) **EP 2 595 683 B1**
(45) Date of publication and mention of the grant of the patent: **04.03.2015**
(21) Application number: 11735691.5
(22) Date of filing: 19.07.2011
(51) Int. Cl.: A61M 15/00

(54) **Inhaler**
Inhalator
Inhalateur

(30) Priority: 21.07.2010 US 366263 P
(43) Date of publication of application: 29.05.2013
(73) Proprietor: AstraZeneca AB, 151 85 Södertälje (SE)
(72) Inventor: ARVIDSSON, Lars, Rune, Gustav, S-212 14 Malmö (SE); BAKEWELL, William, Royston Hertfordshire SG8 6EE (GB); BRIANT, John, Philip, Royston Hertfordshire SG8 6EE (GB); CAMPBELL, Patrick, Royston Hertfordshire SG8 6EE (GB); COOKE, Charles Brian Durier, Royston Hertfordshire SG8 6EE (GB); GROOMBRIDGE, Christopher Benjamin James, Royston, Hertfordshire SG8 6EE (GB); JOHN, James, Daniel, Royston Hertfordshire SG8 6EE (GB); LASTOW, Orest, S-212 14 Malmö (SE); PENHALLURICK, Trevor, John, Royston Hertfordshire SG8 6EE (GB); SMARTT, Nicholas, Royston Hertfordshire SG8 6EE (GB); SVENSSON, Sven Mårten Jimmy, S-247 32 Sondra Sandby (SE); ULLBRAND, Björn, S-245-32 Staffanstorp (SE)
(86) International application number: PCT/GB2011/051349
(87) International publication number: WO 2012/010877

(56) References cited:
- WO-A1-2010/042035
- US-A1- 2001 027 790
- US-A1- 2007 181 123
- US-A1- 2009 013 994

## Description

### Technical field

The present invention relates to an inhaler comprising a plurality of sealed medicament compartments in a disc assembly which is rotated to bring individual compartments into registry with a mouthpiece (or nosepiece). The invention also relates to a method of indexing the disc assembly in such an inhaler.

### Background of the Invention

There are different types of inhalers on the market. A pressurized Metered Dose Inhaler (pMDI) releases a fixed dose of substance in aerosol form. A powder inhaler generally releases a dose of powdered substance entrained in an air stream. In a powder inhaler the powder may be provided in a bulk container of the inhaler from which doses of powder are metered for dispensing. As an alternative to a bulk container, powder inhalers may comprise a single compartment or a plurality of compartments for containing one or more discrete doses of powdered substance. Such compartments may take the form of sealed blisters in a blister pack, a flexible strip of sealed cavities or other suitable forms.

It is generally desirable for an inhaler to be as small and discrete for the user as possible. This can be an issue for multi-dose dry powder inhalers, especially those in which the medicament is stored in cavities arranged around a rigid disc. In such inhalers, the diameter of the disc is generally the factor which affects overall size more than anything else. Minimising the components of the inhaler external to the outline of the cavity disc is therefore to be desired in order to minimise the overall profile of the inhaler. For example, US2007/181123 discloses a cavity disc inhaler with both a mouthpiece 6 and an indexing mechanism handle 8 outside the periphery of the cavity disc, creating a bulky structure.

Multi-dose dry powder inhalers tend to be complex devices and yet in many cases also need to be completely disposable. Any aspects of the design of such an inhaler which reduce the total number of parts are therefore highly desirable.

An issue with the design of a multi-dose inhaler using a disc of medicament cavities is the communication between the air flow path which crosses the disc and the flow path through the mouthpiece, since the disc is normally movable with respect to the mouthpiece. The normal solution would be to create a seal between the disc and mouthpiece, since leakage of air into the mouthpiece flow channel would create unpredictability in the overall flow resistance of the inhaler and in the proportion of the overall flow which entrains medicament from the disc cavity. However, this would involve creating a seal between components which move relative to one another, introducing complexity into the device.

It is often necessary for a bypass flow path to be provided in an inhaler of this type, that is to say a flow path which does not pass the medicament cavity. The main flow path in such an inhaler will normally have been optimised to entrain powder from a cavity and this may mean that it has a relatively high flow resistance. The overall flow resistance of the inhaler may be reduced to an acceptable level by the use of a bypass flow path.

In US2007/181123, a disc type dry powder inhaler is described in which a bypass channel is incorporated in the disc structure, introducing a degree of complexity to the structure of the disc assembly and increasing the component count compared to a system with no bypass channel. US2007/181123 shows a mouthpiece located beyond the disc outer periphery; it is not stated whether there is a seal between the disc and mouthpiece.

The inventors of the present invention have sought to reduce complexity by providing a bypass flow channel, with dimensions having a high degree of precision, between the edge of the disc and the mouthpiece. Other advantages are achieved by this arrangement as discussed below.

Another possible way of providing bypass air flow which is disclosed in the prior art is to have a separate channel or channels external to the disc, e.g. in the sides of a mouthpiece. For example, EP1106196A1 discloses such an inhaler. This arrangement is not preferred, especially in a breath-actuated inhaler (i.e. an inhaler where the medicament cavity is opened automatically when a user inhales), where it is desirable for all air flow to interact with the breath triggering mechanism upstream of the medicament cavity.

WO 2010/042035 discloses an inhaler according to the preamble of claim 1.

### Summary of the Invention

An object of the present invention is to avoid drawbacks associated with some prior inhalers. This and other objects, which will become apparent in the following, are accomplished by the inhaler and the methods defined in the accompanying claims.

According to the invention, an inhaler comprises:
- a housing and a mouthpiece mounted on the housing (directly or indirectly), the mouthpiece having an inhalation channel,
- a disc shaped assembly comprising a plurality of compartments containing medicament, the disc assembly being rotatably mounted on the housing (directly or indirectly) to allow medicament-containing compartments to be brought sequentially into registry with the mouthpiece, the mouthpiece being located beyond the outer periphery of the disc assembly and being in communication with the disc assembly via an aperture in the housing,
- an indexing mechanism associated with the disc assembly for incrementally advancing the disc assembly,
- wherein the mouthpiece is movably mounted with respect to the housing and the mouthpiece forms part of the indexing mechanism.

Having the mouthpiece as part of the indexing mechanism reduces the number of components in the device. Furthermore, it avoids having a further component which may have to be located outside the disc assembly, which would tend to increase the profile of the device. Preferably, the mouthpiece is movable in a radial or substantially radial direction with respect to the disc assembly.

Preferably, the mouthpiece is a brake, being movable between (i) a braking position in which it is engaged with with disc assembly and the disc assembly is prevented from rotating and (ii) a released position in which it is disengaged from the disc assembly and the disc assembly is able to be advanced. The exterior periphery of the disc assembly is the most appropriate place for a brake to act since more surface is available if it is a friction brake and, whether or not it is a friction brake, the forces on the brake at the outer periphery of the disc will be lower for a given braking effect.

Preferably, the mouthpiece includes a spacing member which extends towards the disc assembly and is engaged with a notch in the disc assembly when in the braking position. This system provides a more positive braking effect than a friction brake, with a lower radial force exerted on the disc by the brake. It also allows a well-defined spacing to be created between the mouthpiece brake and the disc assembly, which spacing may act as a bypass air channel.

It would be possible to have a bypass channel between the disc assembly and the mouthpiece and to have a separate brake acting on the outer periphery of the disc assembly, but this could give rise to difficulties in setting the spacing between the mouthpiece and disc assembly with sufficient precision. A brake acting on the disc assembly could take up tolerance in the disc assembly mounting so that the disc assembly bears on its mounting, or alternatively take up tolerance in a spaced mouthpiece on the other side of the disc assembly. One of these must be chosen, with the result that either the precision of the position of the disc assembly in the housing is compromised or alternatively the precision of the bypass channel dimensions. Compromising the precision of the bypass channel dimensions may cause unpredictability in the flow resistance of the inhaler which is undesirable; compromising the precision of the disc assembly mounting may affect the disc indexing mechanism and so is also undesirable. Using the mouthpiece as the brake solves this problem: the mouthpiece brake (when engaged, which is most of the time) takes up tolerance in the disc assembly mounting, whilst at the same time precisely setting the dimensions of the bypass flow channel.

In use, a composite air flow may be created in the channel, air entering from the bypass channel flowing nearer to the walls of the inhalation channel than air flow entering from a medicament compartment of the disc assembly.

Preferably, the mouthpiece and an outer cover component of the inhaler have cooperating cam surfaces such that relative rotation of the housing and outer cover component moves the mouthpiece between the braking position and the released position. This allows for control of the mouthpiece brake movement with a minimal component count.

The cam surface of the mouthpiece is preferably a projection (cam follower) associated with a spring which, in the braking position, acts to bias the mouthpiece spacing member against the disc assembly. The spring allows tolerance in the disc assembly mounting to be taken up, whilst still ensuring that the spacing member is engaged securely in the notch and the spacing is fully defined.

Preferably, in the braking position, the mouthpiece and the disc assembly define a bypass air channel. This avoids the need for a seal between the disc and mouthpiece, saving on components and complexity, particularly since any seal at this point would be required also to accommodate tolerances in the mounting.

Preferably, a dimension of the bypass air channel is determined by the spacing member in engagement with the disc assembly.

In a preferred embodiment,
- the inhalation channel extends through the mouthpiece and has an inhalation channel inlet,
- the disc shaped assembly comprises a plurality of compartments containing medicament and a plurality of respective medicament outlets, the disc assembly being rotatably mounted to the housing to allow the medicament outlets to be brought sequentially into registry with the mouthpiece,
- the said inhalation channel inlet being spaced from a said medicament outlet with which the mouthpiece is in registry, such that a bypass air channel is defined between the medicament outlet and the inhalation channel inlet.

This arrangement provides a bypass channel at a very low level of complexity.

Preferably, the bypass air channel extends around at least 80%, more preferably 100% of a periphery of the inhalation channel inlet. Having the bypass channel surrounding or substantially surrounding the inlet to the inhalation channel means that air which is not laden with particles may form a "sheath" around a central core of particle-laden air. This may have the effect of reducing deposition of particles on the interior of the inhalation channel. Deposition of particles can be a problem in dry powder inhalers because it introduces uncertainty into the dose received by the patient. Furthermore, it is possible that deposited powder from previous doses may become dislodged during a subsequent inhalation, causing the patient to receive an increased dose.

Preferably, the inhaler mouthpiece includes a spacing member or peg which extends towards the disc and is engagable with the disc in order to define a dimension of the bypass air channel.

A flexible seal member may be provided, forming a sealing contact between the mouthpiece and housing, the seal member being able to accommodate movement of the mouthpiece to bring the spacing member into and out of contact with the disc assembly without breaking the sealing contact between the mouthpiece and the housing.

Preferably, secondary bypass air channels extend through the mouthpiece. The inhaler is preferably breath-actuated in the sense that a user's inhalation triggers opening of a medicament cavity as well as entraining of powder. In a breath-actuated inhaler, it is desirable for the entire air flow on inhalation (including any bypass flow) to act on the breath actuation mechanism. The possible option of having a bypass air channel completely external to the disc assembly, e.g. provided by side apertures in the mouthpiece, is therefore undesirable in a breath-actuated inhaler since the bypass flow would not act on the breath actuation mechanism. The medicament in the inhaler may contain various active ingredients. The active ingredient may be selected from any therapeutic or diagnostic agent. For example, the active ingredient may be an antiallergic, a bronchodilator (e.g. a beta2-adrenoceptor agonist or a muscarinic antagonist), a bronchoconstrictor, a pulmonary lung surfactant, an analgesic, an antibiotic, a mast cell inhibitor, an antihistamine, an anti-inflammatory, an antineoplastic, an anaesthetic, an antitubercular, an imaging agent, a cardiovascular agent, an enzyme, a steroid, genetic material, a viral vector, an antisense agent, a protein, a peptide, a non-steroidal glucocorticoid Receptor (GR Receptor) agonist, an antioxidant, a chemokine antagonist (e.g. a CCR1 antagonist), a corticosteroid, a CRTh2 antagonist, a DP1 antagonist, an Histone Deacetylase Inducer, an IKK2 inhibitor, a COX inhibitor, a lipoxygenase inhibitor, a leukotriene receptor antagonist, an MPO inhibitor, a p38 inhibitor, a PDE inhibitor, a PPARγ agonist, a protease inhibitor, a statin, a thromboxane antagonist, a vasodilator, an ENAC blocker (Epithelial Sodium-channel blocker) and combinations thereof.

Examples of specific active ingredients that can be incorporated in the inhaler include:
(i) antioxidants:- Allopurinol, Erdosteine, Mannitol, N-acetyl cysteine choline ester, N-acetyl cysteine ethyl ester, N-Acetylcysteine, N-Acetylcysteine amide and Niacin;
(ii) chemokine antagonists:- BX471 ((2R)-1-[[2-[(aminocarbonyl)amino]-4-chlorophenoxy]acetyl]-4-[(4-fluorophenyl)methyl]-2-methylpiperazine monohydrochloride), CCX634, *N*-{2-[((2*S*)-3-{[1-(4-chlorobenzyl)piperidin-4-yl]amino}-2-hydroxy-2-methylpropyl)oxy]-4-hydroxyphenyl}acetamide (see WO 2003/051839), and 2-{2-Chloro-5-{[(2S)-3-(5-chloro-1'H,3H-spiro[1-benzofuran-2,4'-piperidin]-1'-yl)-2-hydroxypropyl]oxy}-4-[(methylamino)carbonyl]phenoxy}-2-methylpropanoic acid (see WO 2008/010765), 656933 (N-(2-bromophenyl)-N'-(4-cyano-1H-1,2,3-benzotriazol-7-yl)urea), 766994 (4-({[({[(2R)-4-(3,4-dichlorobenzyl)morpholin-2-yl]methyl}amino)carbonyl]-amino}methyl)benzamide), CCX-282, CCX-915, Cyanovirin N, E-921, INCB-003284, INCB-9471, Maraviroc, MLN-3701, MLN-3897, T-487 (N-{1-[3-(4-ethoxyphenyl)-4-oxo-3,4-dihydropyrido [2,3-d]pyrimidin-2-yl]ethyl}-N-(pyridin-3-ylmethyl)-2-[4-(trifluoromethoxy)phenyl]acetamide) and Vicriviroc
(iii) Corticosteroids: -Alclometasone dipropionate, Amelometasone, Beclomethasone dipropionate, Budesonide, Butixocort propionate, Ciclesonide, Clobetasol propionate, Desisobutyrylciclesonide, Etiprednol dicloacetate, Fluocinolone acetonide, Fluticasone Furoate, Fluticasone propionate, Loteprednol etabonate (topical) and Mometasone furoate.
(iv) DP1 antagonists:- L888839 and MK0525;
(v) Histone deacetylase inducers:- ADC4022, Aminophylline, a Methylxanthine or Theophylline;
(vi) IKK2 inhibitors:- 2-{[2-(2-Methylamino-pyrimidin-4-yl)-1H-indole-5-carbonyl]-amino}-3-(phenyl-pyridin-2-yl-amino)-propionic acid;
(vii) COX inhibitors:- Celecoxib, Diclofenac sodium, Etodolac, Ibuprofen, Indomethacin, Meloxicam, Nimesulide, OC1768, OC2125, OC2184, OC499, OCD9101, Parecoxib sodium, Piceatannol, Piroxicam, Rofecoxib and Valdecoxib;
(viii) Lipoxygenase inhibitors:- Ajulemic acid, Darbufelone, Darbufelone mesilate, Dexibuprofen lysine (monohydrate), Etalocib sodium, Licofelone, Linazolast, Lonapalene, Masoprocol, MN-001, Tepoxalin, UCB-35440, Veliflapon, ZD-2138, ZD-4007 and Zileuton ((±)-1-(1-Benzo[b]thien-2-ylethyl)-1-hydroxyurea);
(ix) Leukotriene receptor antagonists:- Ablukast, Iralukast (CGP 45715A), Montelukast, Montelukast sodium, Ontazolast, Pranlukast, Pranlukast hydrate (mono Na salt), Verlukast (MK-679) and Zafirlukast;
(x) MPO Inhibitors:- Hydroxamic acid derivative (N-(4-chloro-2-methyl-phenyl)-4-phenyl-4-[[(4-propan-2-ylphenyl)sulfonylamino]methyl]piperidine-1-carboxamide), Piceatannol and Resveratrol;
(xi) Beta2-adrenoceptor agonists:- metaproterenol, isoproterenol, isoprenaline, albuterol, salbutamol (e.g. as sulphate), formoterol (e.g. as fumarate), salmeterol (e.g. as xinafoate), terbutaline, orciprenaline, bitolterol (e.g. as mesylate), pirbuterol, indacaterol, salmeterol (e.g. as xinafoate), bambuterol (e.g. as hydrochloride), carmoterol, indacaterol (CAS no 312753-06-3; QAB-149), formanilide derivatives e.g. 3-(4-{[6-({(2R)-2-[3-(formylamino)-4-hydroxyphenyl]-2-hydroxyethyl}amino)hexyl]oxy}-butyl)-benzenesulfonamide; 3-(4-{[6-({(2R)-2-hydroxy-2-[4-hydroxy-3-(hydroxymethyl)phenyl]ethyl}amino)-hexyl]oxy}butyl)benzenesulfonamide; GSK 159797, GSK 159802, GSK 597901, GSK 642444, GSK 678007; and a compound selected from *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide, *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(3-chlorophenyl)ethoxy]propanamide, 7-[(1*R*)-2-({2-[(3-{[2-(2-Chlorophenyl)ethyl]amino}propyl)thio]ethyl}amino)-1-hydroxyethyl]-4-hydroxy-1,3-benzothiazol-2(3*H*)-one, and *N*-Cyclohexyl-*N*³-[2-(3-fluorophenyl)ethyl]-*N*-(2-{[2-(4-hydroxy-2-oxo-2,3-dihydro-1,3-benzothiazol-7-yl)ethyl]amino}ethyl)-β-alaninamide or a pharmaceutically acceptable salt thereof (e.g. wherein the counter ion is hydrochloride (for example a monohydrochloride or a dihydrochloride), hydrobromide (for example a monohydrobromide or a dihydrobromide), fumarate, methanesulphonate, ethanesulphonate, benzenesulphonate, 2,5-dichlorobenzenesulphonate, *p-*toluenesulphonate, napadisylate (naphthalene-1,5-disulfonate or naphthalene-1-(sulfonic acid)-5-sulfonate), edisylate (ethane-1,2-disulfonate or ethane-1-(sulfonic acid)-2-sulfonate), D-mandelate, L-mandelate, cinnamate or benzoate.)
(xii) Muscarinic antagonists:- Aclidinium bromide, Glycopyrrolate (such as R,R-, R,S-, S,R-, or S,S-glycopyrronium bromide), Oxitropium bromide, Pirenzepine, telenzepine, Tiotropium bromide, 3(R)-1-phenethyl-3-(9H-xanthene-9-carbonyloxy)-1-azoniabicyclo[2.2.2]octane bromide, (3R)-3-[(2S)-2-cyclopentyl-2-hydroxy-2-thien-2-ylacetoxy]-1-(2-phenoxyethyl)-1-azoniabicyclo[2.2.2]actane bromide, a quaternary salt (such as [2-((R)-Cyclohexyl-hydroxy-phenyl-methyl)-oxazol-5-ylmethyl]-dimethyl-(3-phenoxypropyl)-ammonium salt, [2-(4-Chloro-benzyloxy)-ethyl]-[2-((R)-cyclohexyl-hydroxy-phenyl-methyl)-oxazol-5-ylmethyl]- dimethyl-ammonium salt and (R)-1-[2-(4-Fluoro-phenyl)-ethyl]-3-((*S*)-2-phenyl-2-piperidin-1-yl-propionyloxy)-1-azonia-bicyclo[2.2.2]octane salt wherein the counter-ion is, for example, chloride, bromide, sulfate, methanesulfonate, benzenesulfonate (besylate), toluenesulfonate (tosylate), napthalenebissulfonate (napadisylate or heminapadisylate), phosphate, acetate, citrate, lactate, tartrate, mesylate, maleate, fumarate or succinate)
(xiii) p38 Inhibitors:- 681323, 856553, AMG548 (2-[[(2S)-2-amino-3-phenylpropyl]amino]-3-methyl-5-(2-naphthalenyl)-6-(4-pyridinyl)-4(3H)-pyrimidinone), Array-797, AZD6703, Doramapimod, KC-706, PH 797804, R1503, SC-80036, SCIO469, 6-chloro-5-[[(2*S*,5*R*)-4-[(4-fluorophenyl)methyl]-2,5-domethyl-1-piperazinyl]carbonyl]-*N*,*N*,1-trimethyl-a-oxo-1*H*-indole-3-acetamide, VX702 and VX745 (5-(2,6-dichlorophenyl)-2-(phenylthio)-6H-pyrimido[1,6-b]pyridazin-6-one);
(xiv) PDE Inhibitors:- 256066, Arofylline (3-(4-chlorophenyl)-3,7-dihydro-1-propyl-1H-Purine-2,6-dione), AWD 12-281 (N-(3,5-dichloro-4-pyridinyl)-1-[(4-fluorophenyl)methyl]-5-hydroxy-α-oxo-1H-indole-3-acetamide), BAY19-8004 (Bayer), CDC-801 (Calgene), Celgene compound ((βR)-β-(3,4-dimethoxyphenyl)-1,3-dihydro-1-oxo-2H-isoindole-2-propanamide), Cilomilast (cis-4-cyano-4-[3-(cyclopentyloxy)-4-methoxyphenyl]-cyclohexanecarboxylic acid), 2-(3,5-dichloro-4-pyridinyl)-1-(7-methoxyspiro[1,3-benzodioxole-2,1'-cyclopentan]-4-yl)ethanone (CAS number 185406-34-2)), (2-(3,4-difluorophenoxy)-5-fluoro-N-[cis-4-[(2-hydroxy-5-methylbenzoyl)amino]cyclohexyl]-)-3-pyridinecarboxamide), (2-(3,4-difluorophenoxy)-5-fluoro-N-[cis-4-[[2-hydroxy-5-(hydroxyrnethyl)benzoyl]amino]cyclohexyl]-3-pyridinecarboxamide,), CT2820, GPD-1116, Ibudilast, IC 485, KF 31334, KW-4490, Lirimilast ([2-(2,4-dichlorobenzoyl)-6-[(methylsulfonyl)oxy]-3-benzofuranyl])-urea), (N-cyclopropyl-1,4-dihydro-4-oxo-1-[3-(3-pyridinylethynyl)phenyl]-)-1,8-naphthyridine-3-carboxamide), (N-(3,5-dichloro-4-pyridinyl)-4-(difluoromethoxy)-8-[(methylsulfonyl)amino])-1-dibenzofurancarboxamide), ONO6126, ORG 20241 (4-(3,4-dimethoxyphenyl)-N-hydroxy-)-2-thiazolecarboximidamide), PD189659/PD168787 (Parke-Davis), Pentoxifylline (3,7-dihydro-3,7-dimethyl-1-(5-oxohexyl)-)-1H-purine-2,6-dione), compound (5-fluoro-N-[4-[(2-hydroxy-4-methyl-benzoyl)amino]cyclohexyl]-2-(thian-4-yloxy)pyridine-3-carboxamide), Piclamilast (3-(cyclopentyloxy)-N-(3,5-dichloro-4-pyridinyl)-4-methoxy-benzamide), PLX-369 (WO 2006026754), Roflumilast (3-(cyclopropylmethoxy)-N-(3,5-dichloro-4-pyridinyl)-4-(difluoromethoxy)benzamide), SCH 351591 (N-(3,5-dichloro-1-oxido-4-pyridinyl)-8-methoxy-2-(trifluoromethyl)-5-quinolinecarboxamide), SeICID(TM) CC-10004 (Calgene), T-440 (Tanabe), Tetomilast (6-[2-(3,4-diethoxyphenyl)-4-thiazolyl]-2-pyridinecarboxylic acid), Tofimilast (9-cyclopentyl-7-ethyl-6,9-dihydro-3-(2-thienyl)-5H-pyrazolo[3,4-c]-1,2,4-triazolo[4,3-a]pyridine), TPI 1100, UCB 101333-3 (N,2-dicyclopropyl-6-(hexahydro-1H-azepin-1-yl)-5-methyl-4-pyrimidinamine), V-11294A (Napp), VM554/VM565 (Vernalis), and Zardaverine (6-[4-(difluoromethoxy)-3-methoxyphenyl]-3(2H)-pyridazinone).
(xv) PDE5 Inhibitors:- Gamma-glutamyl[s-(2-iodobenzyl)cysteinyl]glycine, Tadalafil, Vardenafil, sildenafil, 4-phenyl-methylamino-6-chloro-2-(1-imidazolyl)-quinazoline, 4-phenyl-methylamino-6-chloro-2-(3-pyridyl)-quinazoline, 1,3-dimethyl-6-(2-propoxy-5-methanesulphonylamidophenyl)-1,5-dihydropyrazolo[3,4-d]pyrimidin-4-one and 1-cyclopentyl-3-ethyl-6-(3-ethoxy-4-pyridyl)-pyrazolo[3,4-d]pyrimidin-4-one;
(xvi) PPARγ agonists:- Pioglitazone, Pioglitazone hydrochloride, Rosiglitazone Maleate, Rosiglitazone Maleate ((-)-enantiomer, free base), Rosiglitazone maleate/Metformin hydrochloride and Tesaglitizar;
(xvii) Protease Inhibitors:- Alpha1-antitrypsin proteinase Inhibitor, EPI-HNE4, UT-77, ZD-0892, DPC-333, Sch-709156 and Doxycycline;
(xviii) Statins:- Atorvastatin, Lovastatin, Pravastatin, Rosuvastatin and Simvastatin
(xix) Thromboxane Antagonists: Ramatroban and Seratrodast;
(xx) Vasodilators:- A-306552, Ambrisentan, Avosentan, BMS-248360, BMS-346567, BMS-465149, BMS-509701, Bosentan, BSF-302146 (Ambrisentan), Calcitonin Gene-related Peptide, Daglutril, Darusentan, Fandosentan potassium, Fasudil, Iloprost, KC-12615 (Daglutril), KC-12792 2AB (Daglutril), Liposomal treprostinil, PS-433540, Sitaxsentan sodium, Sodium Ferulate, TBC-11241 (Sitaxsentan), TBC-3214 (N-(2-acetyl-4,6-dimethylphenyl)-3-[[(4-chloro-3-methyl-5-isoxazolyl)amino]sulfonyl]-2-thiophenecarboxamide), TBC-3711, Trapidil, Treprostinil diethanolamine and Treprostinil sodium;
(xxi) ENACs:- Amiloride, Benzamil, Triamterene, 552-02, PSA14984, PSA25569, PSA23682 and AER002.

The inhaler may contain a combination of two or more active ingredients, for example a combination of two or more of the specific active ingredients listed in (i) to (xxi) herein above.

In one embodiment the inhaler contains an active ingredient selected from mometasone, ipratropium bromide, tiotropium and salts thereof, salemeterol, fluticasone propionate, beclomethasone dipropionate, reproterol, clenbuterol, rofleponide and salts, nedocromil, sodium cromoglycate, flunisolide, budesonide, formoterol fumarate dihydrate, terbutaline, terbutaline sulphate, salbutamol base and sulphate, fenoterol, 3-[2-(4-Hydroxy-2-oxo-3H-1,3-benzothiazol-7-yl)ethylamino]-N-[2-[2-(4-methylphenyl)ethoxy]ethyl]propane-sulphonamide, hydrochloride, indacaterol, aclidinium bromide, *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide or a pharmaceutically acceptable salt thereof (e.g. dihydrobromide); *N*-Cyclohexyl-*N*³-[2-(3-fluorophenyl)ethyl]-*N*-(2-{[2-(4-hydroxy-2-oxo-2,3-dihydro-1,3-benzothiazol-7-yl)ethyl]amino}ethyl)-β-alaninamide or a pharmaceutically acceptable salt thereof (e.g. diD-mandelate); a [2-(4-Chloro-benzyloxy)-ethyl]-[2-((R)-cyclohexyl-hydroxy-phenylmethyl)-oxazol-5-ylmethyl]- dimethyl-ammonium salt (e.g. hemi-naphthalene-1,5-disulfonate); a (*R*)-1-[2-(4-Fluoro-phenyl)-ethyl]-3-((*S*)-2-phenyl-2-piperidin-1-yl-propionyloxy)-1-azonia-bicyclo[2.2.2]octane salt (e.g. bromide or toluenesulfonate); or a combination of any two or more thereof.

Specific combinations of active ingredients which may be incorporated in the inhaler include:-
(a) formoterol (e.g. as fumarate) and budesonide;
(b) formoterol (e.g. as fumarate) and fluticasone;
(c) *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide or a pharmaceutically acceptable salt thereof (e.g. dihydrobromide) and a [2-(4-Chloro-benzyloxy)-ethyl]-[2-((R)-cyclohexyl-hydroxy-phenyl-methyl)-oxazol-5-ylmethyl]-dimethyl-ammonium salt (e.g. hemi-naphthalene-1,5-disulfonate);
(d) *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide or a pharmaceutically acceptable salt thereof (e.g. dihydrobromide) and a (*R*)-1-[2-(4-Fluoro-phenyl)-ethyl]-3-((*S*)-2-phenyl-2-piperidin-1-yl-propionyloxy)-1-azonia-bicyclo[2.2.2]octane salt (e.g. bromide or toluenesulfonate);
(e) *N*-Cyclohexyl-*N*³-[2-(3-fluorophcnyl)ethyl]-*N*-(2-{[2-(4-hydroxy-2-oxo-2,3-dihydro-1,3-benzothiazol-7-yl)ethyl]amino}ethyl)-(3-alaninamide or a pharmaceutically acceptable salt thereof (e.g. di-D-mandelate) and [2-(4-Chloro-benzyloxy)-ethyl]-[2-((R)-cyclohexyl-hydroxy-phenyl-methyl)-oxazol-5-ylmethyl]-dimethyl-ammonium salt (e.g. hemi-naphthalene-1,5-disulfonate);
*N*-Cyclohexyl-*N*³-[2-(3-fluorophenyl)ethyl]-*N*-(2-{[2-(4-hydroxy-2-oxo-2,3-dihydro-1,3-benzothiazol-7-yl)ethyl]amino}ethyl)-β-alaninamide or a pharmaceutically acceptable salt thereof (e.g. di-D-mandelate) and a (*R*)-1-[2-(4-Fluoro-phenyl)-ethyl]-3-((*S*)-2-phenyl-2-piperidin-1-yl-propionyloxy)-1-azonia-bicyclo[2.2.2]octane salt (e.g. bromide or toluenesulfonate).

### Brief description of the drawings

Fig. 1 is a perspective view from above of an inhaler according to at least one example embodiment of the invention, in an open configuration;
Fig. 2 is a perspective view from above of the inhaler of Figure 1, in a closed configuration;
Fig. 3 is a perspective view from below of the inhaler of Figure 1, in an open configuration;
Fig. 4 is a perspective view from below of the inhaler of Figure 1, with an outer casing component removed;
Fig. 5 is an exploded perspective view of the inhaler of Figure 1;
Fig. 6 is a cross sectional view of selected components of the inhaler of Figure 1, in a primed condition;
Fig. 7 is a cross sectional view of selected components of the inhaler of Figure 1, in a fired condition;
Fig. 8 is a perspective view from below of a mouthpiece and upper outer casing component of the inhaler of Figure 1;
Fig. 9 is a plan view from below of the cavity disc and indexing mechanism of the inhaler of Figure 1;
Fig. 10 is a perspective view of part of the indexing mechanism and actuator of the inhaler of Figure 1;
Fig. 11 is a perspective view of the mouthpiece of the inhaler of Figure 1;
Fig. 12 is a plan view from above of the lower housing, drive member (indexer) and torsion spring; and
Figure 13 is a perspective view of the inhaler of Figure 1 with the lower housing and lower cover separated, and with some components omitted for clarity.

### Detailed description of the drawings

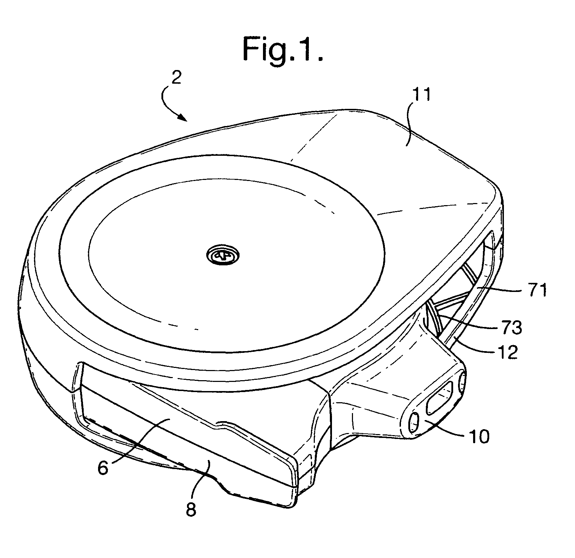

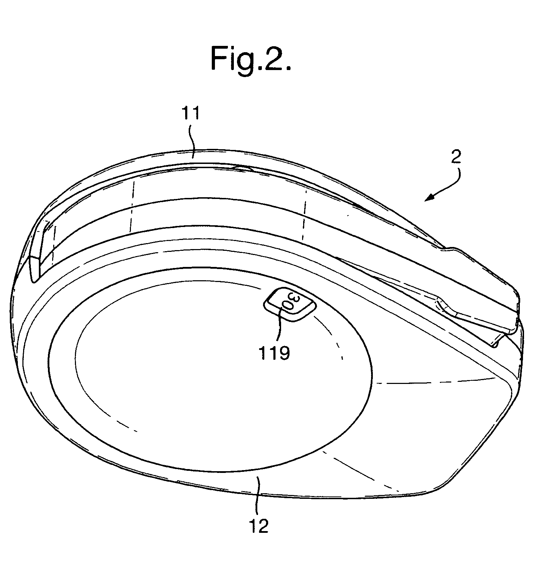

Referring to the Figures, the inhaler 2 comprises a dose dispensing assembly 4 having a general disc configuration, an upper housing portion 6 and a lower housing portion 8, both of 30% glass fibre reinforcement plastic (eg. polybutylene terephtalate, PBT). The inhaler also comprises a mouthpiece assembly, the assembly comprising a mouthpiece member 10 of polypropylene (e.g. Purell HM671T) with a thermoplastic vulcanizated elastomeric mouthpiece seal member 9 which seals the interface between the mouthpiece assembly and the housing. Alternatively, the seal member 9 could for example comprise an elastomeric member such as Santoprene 8281-45MED. The housing is pivotally mounted within a casing comprising upper and lower outer casing components 11, 12 of polycarbonate (e.g. Makrolon 2458); in this way the inhaler can be moved between open and closed configurations. In the open configuration, the mouthpiece is exposed and an external air inlet 71 opened up adjacent the mouthpiece (see e.g. Figure 1). In the closed configuration, the mouthpiece is enclosed within the outer cover 11, 12 and the air inlet 71 shut (see e.g. Figure 2).

The dose dispensing assembly 4 comprises a cavity disc 14 of high density polyethylene (such as Purell GC7260) which has a plurality of cavities 16 formed in one major face of the disc, evenly spaced around the periphery. An alternative material for the dose dispensing assembly 4 is polypropylene (e.g. Purell HM671T). The cavities 16 contain dry powder medicament for inhalation (not shown), and are sealed by a laminated film 18 of aluminium foil and polymer material (referred to as a "foil layer"), thus providing sealed compartments. Above each cavity 16, a respective associated separating element 20 of polypropylene is attached to the upper side of the foil layer 18. Alternatively, the separating element can be formed of high density polyethylene (such as Purell GC7260) particularly when the dose dispensing assembly 4 is polypropylene (e.g. Purell HM671T). The separating elements 20 are attached by any suitable type of bonding, welding, gluing, etc. to the respective part of the foil layer 18. Upwards movement or lifting of a separating element 20 causes the attached part of the foil layer 18 to become separated from the cavity 16. The foil layer 18 has radial cuts between each separating element.

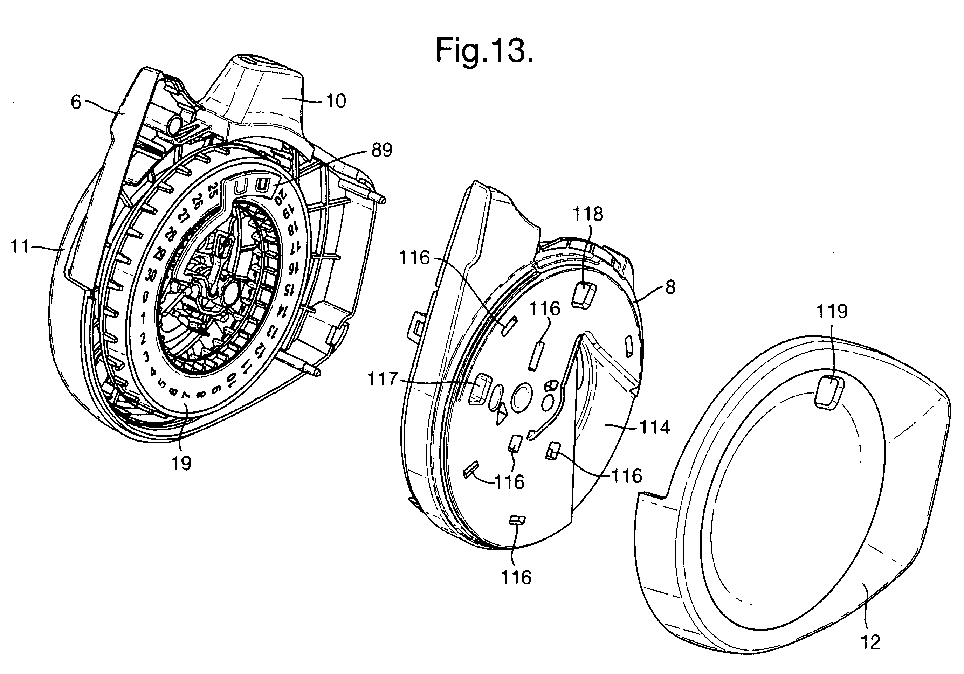

On the opposite face of the cavity disc 14 is a second annular foil layer 19 (see Figure 13) bearing numbers 1-30 corresponding to the medicament cavities 16. Underneath the cavities a space is defined extending around the disc. This space contains a desiccant material (either as molecular sieve or silica gel), and the second foil layer 19 seals the desiccant in this space. In certain states of the inhaler, one of the numbers printed on the second foil are visible though windows in the lower housing portion 8 and lower cover 12. This is explained more fully below.

A circular guide structure 22 of high density polyethylene is provided above the separating elements 20. The guide structure could alternatively be formed of polypropylene (e.g. Purell HM671T). The guide structure 22 comprises a plurality of guide sections 24 divided by vertically extending walls, each guide section 24 being associated with a respective separating element 20. When a separating element 20 is lifted from the cavity disc 14, the associated guide section 24 will guide the upwards movement of the separating element 20. Each guide section 24 is provided with a blade spring 26 as part of the moulding. The blade spring bears downwardly on the top of the respective separating element 20. After a separating element 20 has been lifted and medicament in the opened cavity 16 has been entrained in the inhalation airflow and the separating element 20 has returned to the disc 14, the blade spring 26 will keep the lifted separating element 20 in contact with the disc 14 to cover the cavity 16. This will make it difficult for any remaining powder to exit the covered used cavity 16, thus reducing the risk of dose variation which could occur if remaining powder were to be entrained in a following inhalation. It also reduces the risk of remaining powder exiting the cavity 16 and jamming mechanical components in the inhaler or the risk of the separating element creating a rattling noise which would be undesirable for the user.

The vertical walls dividing the circular guide structure 22 into guide sections 24 function as lateral flow path defining elements. Thus, inhalation airflow is prevented from deviating sideways once it reaches the cavity area of the disc 14 and will be led to the mouthpiece 10. An alternative would be to have shorter vertical walls, in which case neighbouring separating elements 20 could have the function of lateral flow path defining elements.

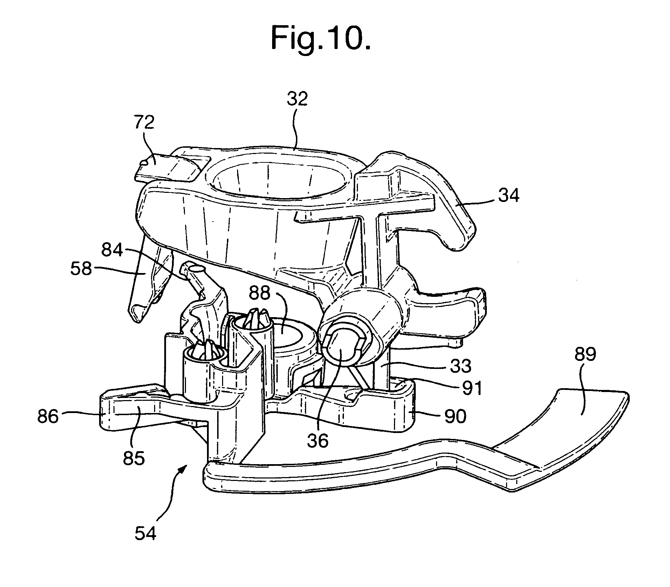

Each separating element 20 has a cavity-covering portion 28 which is in register with a respective cavity 16 in the base. Additionally, each separating element 20 has a centrally projecting portion 30, extending inwardly towards the centre of the disc assembly. An opening mechanism comprising an actuator 32 for lifting the separating elements 20 is provided. The actuator is in the form of a pivotable lever of polyoxymethylene (POM, e.g. Hostaform MT12U01) provided with jaws 34 for gripping the centrally projecting portions 30 of the separating elements 20. The actuator 32 has an energized position in which the jaws 34 are in a lowered position and an unloaded position in which the jaws 34 are in a raised position. The actuator 32 does not rotate with the disc assembly but remains oriented towards the mouthpiece; it is pivotable around the horizontal hinge 36 (see Figure 10) to move between raised (fired) and lowered (energised) positions.

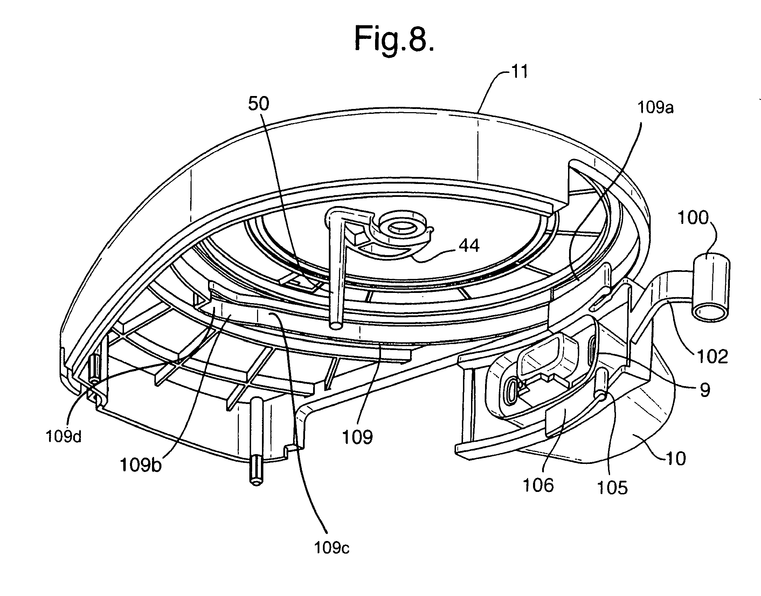

Referring to Figure 8, the upper outer cover component comprises, on its interior surface, a central cam 44, an elongate force transmitting member 50 and a cam track 109. The function of these components is explained below.

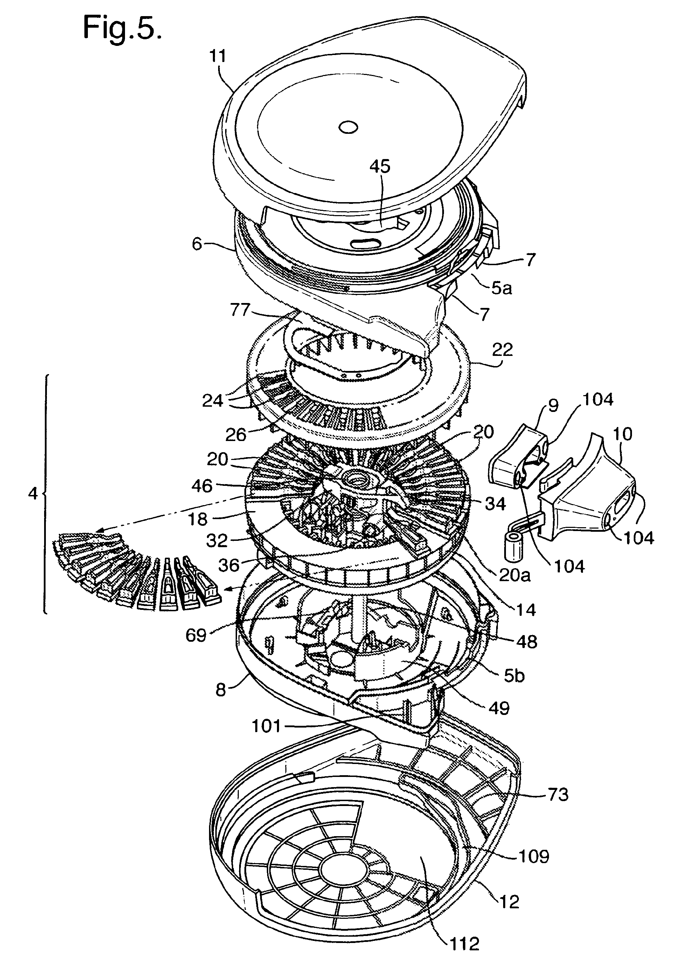

The inhaler housing (together with the mouthpiece assembly) are arranged to pivot with respect to the cover 11, 12 between a closed position in which the mouthpiece 10 is enclosed in the cover and an open position in which the mouthpiece is exposed for use. The central cam 44 engages with the actuator 32 to reset it when the inhaler is closed by rotating the main housing 6, 8 with respect to the outer cover 11, 12. As the cam 44 comes into contact with the jaws 34 of the actuator 32, the actuator 32 will rotate around its pivot 36. The jaws 34 will drop down to the primed or energized position of the actuator 32. The lowering of the jaws 34 will be against the force of a coil spring 46 of stainless steel which is biased to raise the jaws 34 to the unloaded position (although in fact the jaws are not totally unloaded in the raised position). The coil spring 46 is wound around a post 48 projecting upwardly from the lower housing portion 8 and may be seen, for example, in Figures 5, 6 & 7.

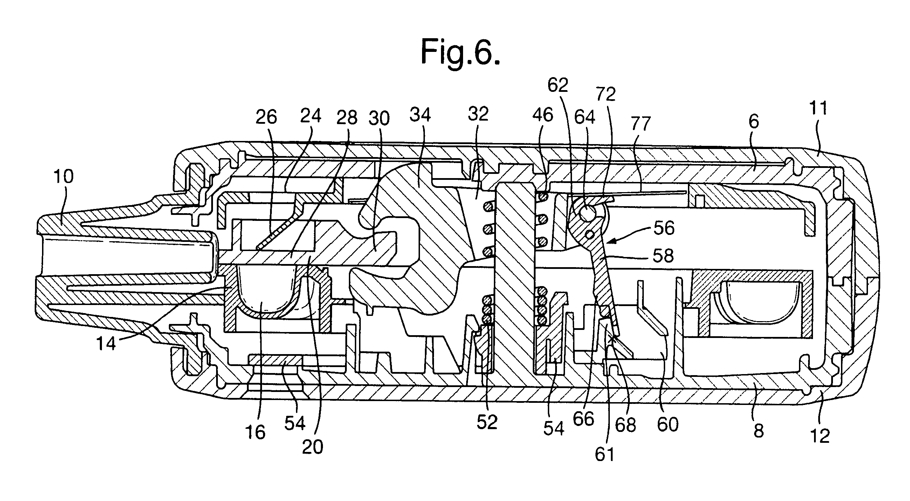

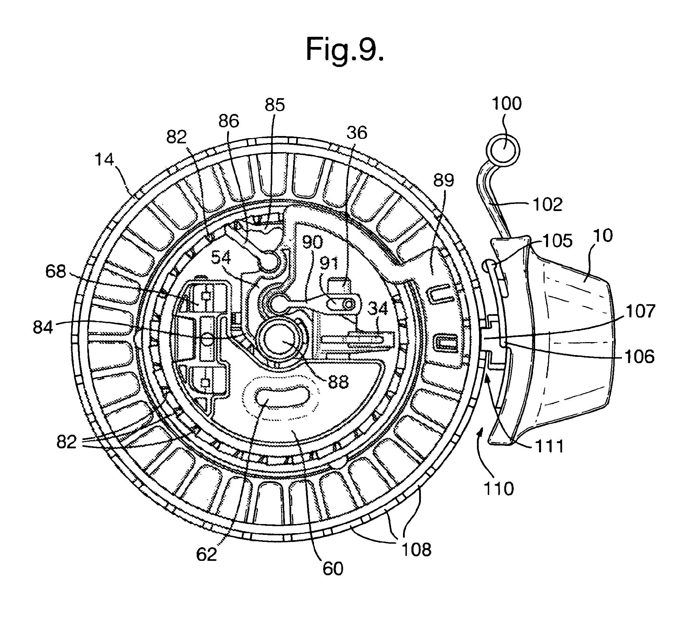

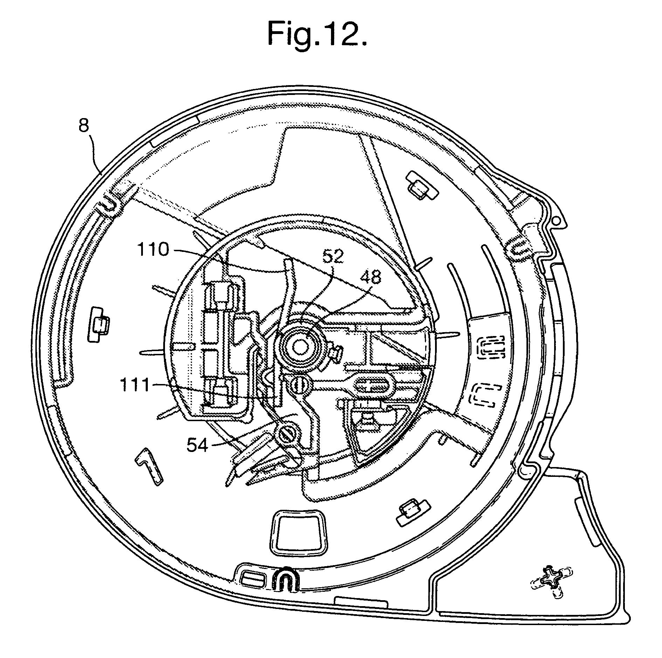

The force transmitting member 50 engages one end 110 of a torsion spring 52 of stainless steel located under the coil spring 46 and around the same post 48 (see Figures 6, 7 and 12). The torsion spring 52 is connected at its other end 111 to a drive member 54 of polyoxymethylene (POM, e.g. Hostaform MT12U01) for rotatingly advancing the cavities 16 by one increment at a time, so as each time to bring an unopened cavity into alignment with the mouthpiece 10. The drive member is best seen in Figures 9 and 12.

A latch 56 is provided to keep the actuator in the energized position. The latch 56 comprises a first element in the form of a prop 58 of polycarbonate (e.g. Makrolon 2458) and a second element in the form of a flap 60 of polyoxymethylene (POM, e.g. Hostaform MT12U01). The prop 58 has a first end portion 62 which is pivotable around a first horizontal pivot 64 on the actuator 32, near the opposite end of the actuator 32 to the jaws 34. The prop 58 has a second end portion 66 adapted to be supported on a shoulder 61 at one end of the flap 60. The flap 60 is pivotable around a second horizontal pivot 68 shown in Figure 6 simply by a cross indicating the axis of the pivot; the structure of the second pivot 68 is partly shown in Figure 5, where part of the support 69 for a corresponding pivot pin on the flap 60 is shown.

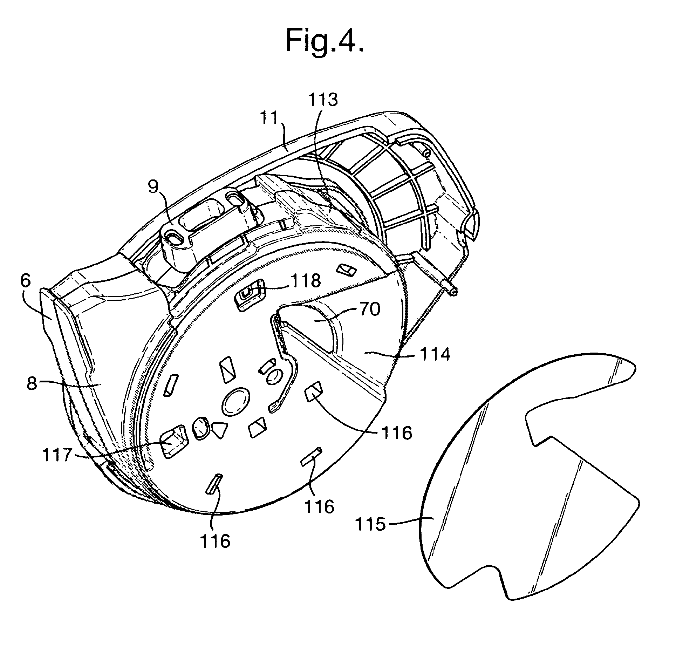

The flap 60 covers a flap valve aperture 70 provided in the lower housing portion 8, which may be seen in Figure 4. Air is allowed to enter the inhaler 2 through the aperture 70 when the user inhales through the mouthpiece 10 (outlet). The incoming air moves the flap, which in turn triggers the actuator 32 to open a cavity so that medicament may be entrained in the air flow. This will be explained in more detail below.

When the inhaler is in the open configuration shown in Figs 1, 3 and 4, air may enter the outer casing through an external air inlet71 between the outer casing and mouthpiece. A first part of an inlet air flow path is thus defined between the the outer cover and a region 113 of the side wall of the housing (see Figure 4).

From the first part of the inlet air flow path, air then passes between the flat internal face of the lower cover 12 and flat external surface of the lower housing 8 to reach the flap valve aperture 70 defined in the lower housing portion 8 (see Figure 4). This second part of the inlet air flow path is defined partly by a portion 112 of the inner surface of the lower outer casing component 12 (see Figure 5) which is kept free from reinforcing ribs which could impede or obstruct flow. The second part of the inlet air flow path is also partly defined by a slightly recessed region 114 of the lower housing 8 leading to the flap valve aperture 70. Figure 4 shows the underside of the lower housing portion 8 which has a number of apertures formed in in it in addition to the flap valve aperture 70. One of these apertures is a dose counter window 117 through which a dose count number printed on the lower foil layer of the cavity disc becomes visible when the inhaler is in a closed configuration. In the closed configuration the dose counter window 117 is in registry with a dual purpose display window 119 in the lower casing member 12, such that the dose count is visible to a user. This is best understood with reference to Figure 13. Figure 2 shows the inhaler from underneath in the closed configuration, with the dose count number displayed in the dual purpose window 119.

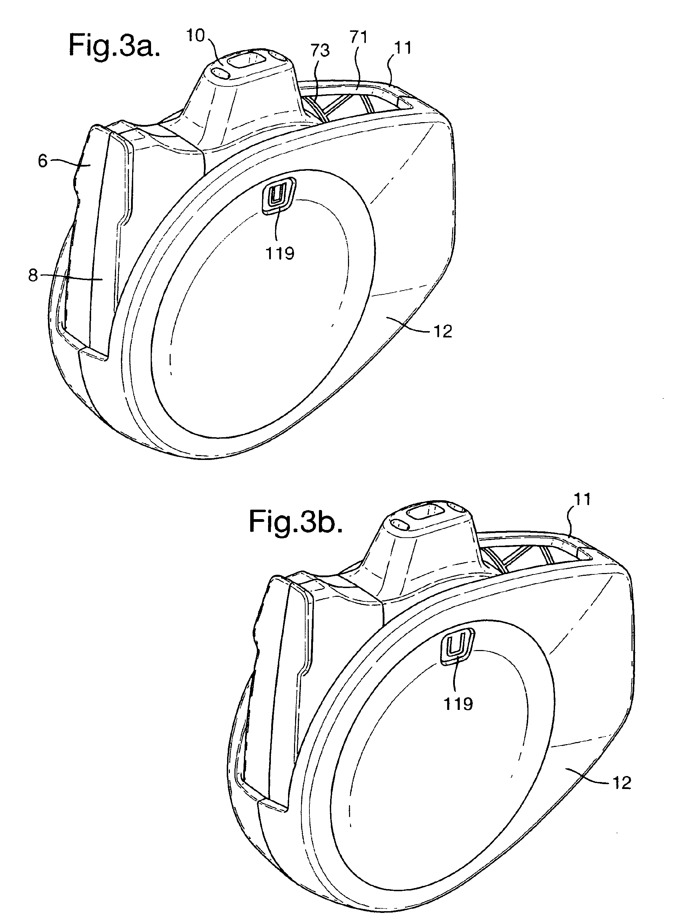

Referring again to Figures 4 and 13, a "ready for use" indicator window 118 is also provided in the underside of the lower housing portion, though which a display flag component 89 on the drive member 54 may be viewed. The ready for use indicator window comes into registry with the dual purpose display window 119 when the inhaler is in an open configuration, allowing the flag 89 to be viewed instead of the dose count number. The flag 89 has two indicia on it, one indicating that the inhaler is ready for use, and the other indicating that the current medicament cavity has been emptied. The position of the display flag changes in dependence on the state of the inhaler, so that the appropriate indicium is visible through the windows 118, 119. The structure and operation of the drive member 54 and display flag 89 will be discussed more fully below, but the states of the "ready for use" indicator may be understood with reference to Figures 3a and 3b which show, respectively, the "ready" and "fired" states of the inhaler.

When the inhaler is in the closed configuration (Figure 2), the mouthpiece assembly 9, 10 is received into the external air inlet 71, closing off the inlet and thereby helping to protect both the mouthpiece and the inlet air flow paths from being contaminated by particles of dirt.

Referring again to Figure 4, a number of apertures 116 are shown in addition to the dose counter and ready for use windows 117, 118. These further apertures 116 are a result of the injection moulding process and are necessary in order for various parts of the inhaler to be moulded in one piece with the lower housing. A transparent membrane 115 is shown separated from the lower housing portion 8. In the assembled inhaler the transparent membrane 115 is secured by adhesive to the lower surface of the lower housing 8 thereby sealing the moulding apertures 116 as well as the windows 117,118 and helping to prevent leakage air flow paths. It is important to minimise leakage air flow paths because they can reduce the air flow in the main air channels of the inhaler when the inhaler is used to values below those needed for correct functioning. They can also cause unpredictability in the air flow.

Because the membrane 115 is transparent, it allows the dose count and ready for use indicia to be viewed through the respective windows 117,118. It is possible to prepare the membrane 115 as a self adhesive film of polymer material, which is simple to assemble to the housing. The entire membrane may be coated with transparent adhesive or, alternatively, the portions of the membrane corresponding to the windows 117,118 may be left free of adhesive.

Fig. 6 is a schematic cross-sectional view of selected details of the inhaler, showing the inhaler in a primed state with the actuator 32 latched in an energized position. The jaws 34 of the actuator 32 have been lowered against the force of the coil spring 46. The dispensing assembly 4 is rotated, after the jaws 34 are lowered, to bring the next unopened cavity 16 into alignment with the mouthpiece 10. The jaws 34 now enclose the centrally projecting portion 30 of a separating element 20 associated with the unopened cavity. The second end portion 66 of the prop 58 is supported by the shoulder 61 of the flap 60. The latch 56 comprising the prop 58 and the flap 60 is now in its first position, in which it latches the actuator 32 in the energized position. The prop 58 is biased into the position shown in Figure 6 by the actuator, under the influence of the coil spring 46. The interface or contact point between the second end portion 66 of the prop 58 and the flap shoulder 61 is located such that the line of action of the force exerted by the prop on the shoulder is on the same side of the second horizontal pivot 68 (the flap pivot) as the portion of the flap 60 covering the aperture 70. In this way, the flap 60 is held in the illustrated lowered position. As long as the flap 60 remains still, the prop 58 is also prevented from moving, keeping the actuator 32 latched in its energized position.

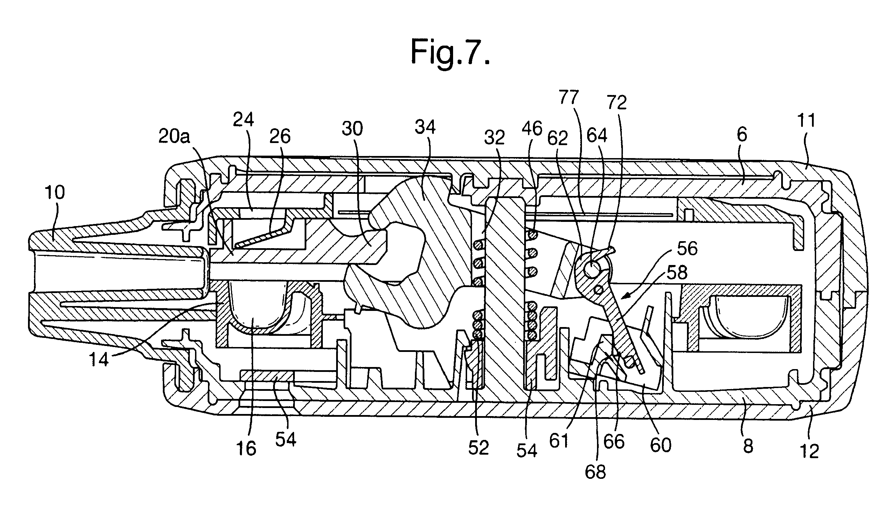

In order to administer a dose, the user inhales, creating a sufficient pressure drop across the flap 60 to raise the flap against the biasing force. This is illustrated in Fig. 7. As the flap 60 is raised and pivoted around the second pivot 68 (clockwise in Fig. 7), the flap shoulder 61 moves to the right in Figures 6 and 7 which results in the prop rolling off the shoulder 61 under the influence of the actuator spring (coil spring 46), the prop pivoting at its upper end around the pivot 64.

During the critical part of the movement, while the prop is being moved by the breath flap against a biasing force, the contact between the prop and shoulder is a rolling contact; the prop and breath flap behave as an over-centre mechanism. This is to minimise friction, which may add to the force required to trigger the mechanism and, more importantly, potentially cause this force to be unpredictable. During this rolling phase of the movement, the line of action of the force exerted on the flap shoulder by the prop moves from the left side of the flap pivot to the right side of the flap pivot. Once this has happened, the flap is no longer biased into the closed position by the prop; pivoting of the flap now continues rapidly, assisted by the force exerted by the prop under the influence of the coil spring 46.

The shape of the shoulder 61 is such that, when the flap reaches a certain angle, the prop will be pushed completely off the shoulder under the influence of the coil spring 46. This last stage of the movement will involve sliding friction, but the movement is driven entirely by the coil spring 46 and is independent of any movement of the breath flap; the coil spring is designed easily to overcome any friction between the prop and the shoulder.

The latch 56 is now in its second position, in which the actuator 32 is free to move to its unloaded position under the influence of the coil spring 46. The actuator 32 will rotate around its pivot 36 and the jaws 34 will be raised. The engaged separating element 20 which is in registry with the jaws 34 is thereby lifted from the cavity disc 14. The portion of the foil layer 18 associated with the cavity 16 which has been opened remains attached to the separating element 20. Figs. 5 and 7 illustrate one separating element 20a in the raised position being raised by the jaws 34 of the actuator 32. On inhalation, air flows across the top of the opened cavity inducing a circulating flow in the cavity which deaggregates medicament powder (not shown) in the cavity and entrains it in a flow of air exiting the inhaler through the mouthpiece 10. Details of the process of emptying the cavity may be found in co-pending applications, numbers PCT/SE2008/051488 (WO2009/082341) and US 61/222209 (from which WO2011/002406 claims priority).

With the prop 58 in the un-latched position shown in Figure 7, the flap 60 is free to return to the lowered position after a dose is dispensed, however the actuator 32 remains in the unloaded position (Fig. 7) until the user primes the inhaler for the next dose.

Closing of the inhaler after use involves rotating the upper and lower casing components 11, 12 (the outer cover) with respect to the rest of the device to achieve the configuration shown in Figure 2. As this is done, the central cam feature 44 (see Figure 8) on the upper outer casing 11 engages with the actuator 32 to lower it and energise the coil spring 46. The central cam 44 accesses the actuator 32 via an aperture 45 in the upper housing portion 6, best seen in Figure 5. In the closed position, the actuator 32 is retained by the central cam 44 so that there is no possibility of a medicament cavity being opened whilst the inhaler is in the closed configuration.

The flap 60 has a protrusion, or flap cam 62 (see Figure 9), on its upper surface which engages with the force transmitting member 50 (see Figure 8) which depends from the upper casing component 12. The interengagement of these features retains the breath flap in the lowered position. The force transmitting member 50 and flap cam 62 remain engaged as the inhaler is opened, until the fully open configuration is reached, or nearly reached, at which point the flap 60 is released. This arrangement is provided to reduce the possibility of the flap being deflected by an inhalation when the inhaler is only partly open, which may result in incorrect functioning of one or more of the other components in the inhaler.

As the inhaler is closed, an indexing mechanism moves the cavity disc around to position an unopened cavity adjacent the mouthpiece 10 and the actuator 32.

The indexing mechanism comprises a drive member or indexer 54 including an integral pawl 85, an additional pawl 86, a display flag 89 and a pulling arm 90 (also known as an indexer link), as well as the torsion spring 52 and the mouthpiece 10.

Figure 9 shows the cavity disc 14 and indexing mechanism from below. The indexer or drive member 54 is shown in detail in Figure 10 together with the additional pawl 86, display flag 89, indexer link 90, actuator 32 and prop 58. The drive member 54 is a single moulding of polyoxymethylene (POM, e.g. Hostaform MT12U01) which comprises an integral pawl 85, prop preventer arm or catch 84, display flag 89 and mounting aperture 88. The drive member 54 is mounted via its mounting aperture 88 to the central post 48 on the lower housing 8 (the housing and post are not shown in Figure 9 but may be seen e.g. in Figure 5). The additional pawl 86 is a separate moulding (e.g. a POM moulding), attached to the indexer 54 by a snap fit pivot connection. Both the integral pawl and additional pawl engage with or between teeth 82 on the internal circumference of the cavity disc 14. The indexer link 90 is also a separate moulding of polypropylene (or alternatively a POM moulding), attached to the indexer 54 by a snap fit pivot connection. The indexer link includes a hole 91 into which projects a peg 33 on the lower side of the actuator 32 (see Figure 10). The functioning of the indexing mechanism will be described more fully below.

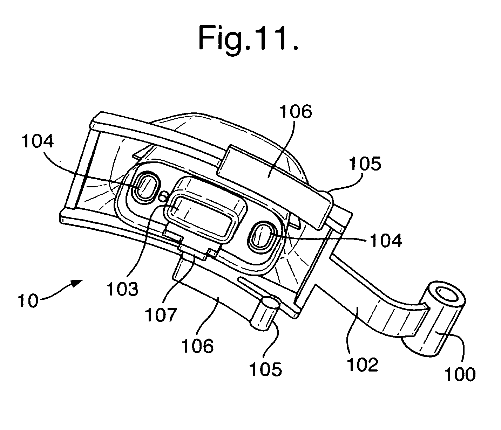

The mouthpiece assembly comprises a mouthpiece member 10 and an elastomeric seal member 9. The mouthpiece member 10 is a single moulding from polypropylene (e.g. Purell HM671T) which comprises a main part and a pivot ring 100 on the end of an arm 102 extending from the main part (see Figure 11). The mouthpiece assembly is pivotally mounted on a spigot 101 on the lower housing portion 8 (see Figure 5) which passes through the pivot ring 100. The main part of the mouthpiece assembly, best seen in Figure 11, comprises an inhalation channel 103 and secondary bypass channels 104 on each side. The secondary bypass channels 104 are partly formed by the mouthpiece member 10 and partly by the seal member 9.

On the side which faces the inhaler housing 6, 8, a leaf spring 106 projects obliquely from each of the upper and lower edges. At the distal end of each leaf spring 106 is a cam follower peg 105. The cam followers 105 and leaf springs 106 are integrally moulded with the mouthpiece member 10. The cam followers 105 and leaf springs 106 engage in cam tracks 109 on the inside surfaces of the respective outer covers / casing components 11, 12. The cam track 109 on the lower casing component can be seen in Figure 5, whilst the cam track 109 on the upper casing component 11 can be seen in Figure 8.

Projecting towards the inhaler housing 6, 8 from underneath the inhalation channel 103 of the mouthpiece member 10 is a spacing member or locating peg 107. On the outside circumference of the cavity disc 14 are notches 108 (see e.g. Figure 9) into which the locating peg 107 can project. In this way, the mouthpiece acts as a brake on the cavity disc to prevent indexing of the disc occurring at the wrong time in the indexing sequence.

During the opening and closing of the inhaler, the cam followers 105 and leaf springs 106 travel along the tracks 109 which control the movement of the mouthpiece towards and away from the cavity disc 14, and hence the engagement and disengagement of the locating peg 107 with the notches 108 of the cavity disc. In Figure 8, the mouthpiece and upper casing component 11 are shown when the inhaler is open; the leaf spring and cam follower are in region 109a of the cam track which brings the mouthpiece towards the housing and cavity disc (not shown) such that the disc is braked. A region 109b at the other end of the cam track 109 can be seen in Figure 8; in the region 109b, the track is further away from the disc assembly and comprises a widened portion 109c and a narrower terminal region 109d. When the spring and follower 106, 105 are moved into this region of the track, in the final stages of closing the inhaler, they enter the widened portion 109c, which leads the cam/spring radially outwardly with respect to the disc, and then finally the proximal end of the leaf spring 106 engages in the narrow terminal portion 109d of the track. The mouthpiece is thereby moved away from the cavity disc and housing and then retained securely in that position, releasing the disc to index. This will be explained more fully below.

The resilience of the leaf spring 106 means that the spacing member or locating peg 107 is resiliently brought to bear against the disc 14 when in the braking position. This allows tolerance in the disc mounting to be taken up. The inner edge of the disc 14 (which should more correctly be called an annulus rather than a disc) bears on a bearing flange 49 (see Figure 5) which is an integral part of the lower housing moulding 8. When the brake is engaged, the inner edge of the disc 14 will be biased against the bearing flange 49 in the region of the mouthpiece.

In this state, a precisely defined spacing 110 exists between the disc assembly and the mouthpiece assembly, through which air may pass on inhalation into the secondary bypass channels 104. Air may also pass through a smaller spacing 111 between the inlet of the inhalation channel 103 and the edge of the disc assembly. These spacings are best seen in Figure 9. It is desirable for the dimensions of these spacing to be as well-defined as possible so that the flow patterns and flow resistance are as well-defined as possible. This is achieved by the spacing member 107 being biased into engagement with the disc 14.

The smaller spacing 111 forms an annular bypass channel around the inhalation channel inlet, which may create a "sheath" flow of air around the main flow of particle-laden air from the disc cavity. Since the bypass air does not have particles of powder entrained in it, it may be able to form a barrier between the drug particles and the wall of the inhalation channel, reducing the deposition of drug particles on the wall.

The wall of the upper and lower housing portions 6, 8 have cutaways 5a, 5b respectively which together form an aperture when the housing is assembled through which air passes into the mouthpiece. The mouthpiece seal member 9 forms a seal against the housing wall around this aperture. Baffles 7 are provided on each side of the cutaway 5a in the upper housing portion 6. The function of these baffles is to extend across the front of the cavities on each side of the cavity which is aligned with the mouthpiece inhalation channel 103. This helps to prevent any stray powder from a used cavity from being entrained in the bypass flow through the secondary bypass channels 104; air entering the bypass channels may do so from underneath the baffles, via the cutaway 5b in the lower housing portion.

After a dose has been dispensed, the user closes the inhaler. Through the rotation of the outer cover or casing components 11, 12 relative to the housing 6, 8, the central cam 44 will urge the actuator 32 to move to its energized position. Thus, the jaws 34 of the actuator 32 will move from the raised unloaded position illustrated in Figure 7 to the lowered energized position illustrated in Figure 6.

Substantially simultaneously with the cam 44 urging the actuator 32 into the energised position, the projecting second force transmitting member 50 on the upper outer casing 12 will urge the indexing mechanism to advance the next cavity 16 to be aligned with the mouthpiece 10. More particularly, the projecting member 50 (see Figure 8), passing through the aperture 45 in the upper housing portion (see Figure 5) engages with the torsion spring 52 to energise it. Figure 12 shows the torsion spring 52 mounted on the central post 48 of the lower housing portion 8. The spring 52 is at rest as shown in Figure 12. A first end 110 of the spring 52 is moved clockwise (as viewed in Figure 12) by the force transmitting member 50. A second end 111 of the spring is engaged with the drive member 54. The energized torsion spring 52 will thus urge the connected drive member 54 to rotate around the central axis provided by the post 48 in order to engage the cavity disc 14 and to thereby cause the disc 14 to rotate so as to bring the next cavity 16 into alignment with the mouthpiece 10. However, the force on the drive member 54 provided by the projecting member 50 via the torsion spring 52 is temporarily counteracted, at least until the actuator 32 has reached its energized position, by the mouthpiece brake arrangement described above. The indexing of the disc is thereby prevented until just before the inhaler is closed (when the leaf springs 106 and cam followers 105 reach region 109b in the cam tracks). This arrangement prevents the mechanism trying to index before the actuator is lowered (in which case the actuator would obstruct indexing). It also avoids the possibility of partial indexing if the cover is partially closed and then opened.

As illustrated in Fig. 9, before the brake is released the pawl 85 of the drive member 54 engages one of a plurality of teeth 82 in the disc 14. The prop preventer 84 is in a preventing position, engaged with the prop 58 to prevent it resting on the flap shoulder 61. Thus, in this state of the inhaler, the actuator cannot become latched in the energized position. This reduces the risk of re-firing from the same cavity 16.

As the brake is released, the drive member 54 will move under the influence of the torsion spring 52 and rotate the disc 14 by one cavity. The additional pawl 86 referred to above prevents the drive member 54 from over-rotating the disc 14, ensuring that the inhaler is indexed only one cavity at a time.

At the upper end of the prop 58 is a position-keeping projection 72 which engages with a steel prop spring 77 (best seen in Figure 5) mounted on the inside face of the upper housing portion 6. The prop spring 77 biases the prop 58 laterally against the shoulder 61 of the flap 60. As the drive member 54 rotates the disc 14 the prop preventer 84 will be removed from the preventing position, thereby allowing the prop 58 to become supported by the flap shoulder 61 and latch the energized actuator. The inhaler is now primed.

As previously described, when the user opens the inhaler and inhales through the mouthpiece 10, the flap 60 is raised so that the prop 58 comes off the flap shoulder 61, thereby unlatching the actuator 32. The actuator 32 will be raised under the influence of the coil spring 46 so that the jaws 34 of the actuator 32 remove the separating element 20 and a portion of the foil layer 18 from the cavity 16 presently aligned with the mouthpiece 10. As can be seen in e.g. Fig. 9, a movable pulling arm or indexer link 90 connects the drive member 54 with the actuator 32. As the actuator 32 and the jaws 34 are raised from the primed to the fired state, the pulling arm / indexer link 90 is moved laterally, shifting the drive member 54 round on the post 48, such that the pawl 85 slips back over one ratchet tooth 82 on the disc. The prop preventer catch 84 will consequently be moved back to its preventing position, in which the prop 58 is prevented from engaging the flap shoulder 61. When the user then closes the inhaler, it will once again become primed, following the sequence described above.

If the user, for some reason, does not close the inhaler fully, the spring 106 and cam follower 105 travelling in the track 109 will not reach its point of release (the region 109b of the cam track 109), and consequently the mouthpiece brake 10 will not be released. This in turn means that there will be no indexing. Furthermore, although the actuator 32 is in its energized position, it will not become latched, as latching can only occur in connection with indexing, as explained above - until the indexer (drive member 54) moves round, the prop preventer 84, which is an integral part of the indexer, will prevent latching. If the user then opens the inhaler again after not fully closing it, the actuator 32 will simply move back to its unloaded position.

The sequence of events on opening and closing the inhaler is set out in Table 1 below.

**Table 1**

| | | |
|---|---|---|
| 1 | Closed state - ready to use | Mouthpiece assembly 9, 10 is enclosed by cover; external air inlet 71 is blocked by the housing. |
| | | Actuator 32 is energised, but held in lower position by central cam 44 on upper outer cover 11 |
| | | Prop 58 is spring biased into position to support actuator 32 but actuator is not held in energised state by prop at this stage. Prop preventer 84 on drive member / indexer 54 is disengaged with prop. |
| | | Brake (mouthpiece assembly) is disengaged from edge of cavity disc. |
| | | Torsion spring 52 is partly energised, biasing disc 14 into correct position. An unused cavity 16 in the disc is aligned with the mouthpiece |
| 2 | Open device | Central cam 44 disengages from actuator 32; actuator now held in energised state by prop 58 resting on flap shoulder 61. |
| | | Mouthpiece is exposed and external air inlet 71 adjacent mouthpiece is opened. |
| | | Brake (mouthpiece) is applied to prevent disc from rotating |
| | | Indexer spring 52 is relaxed as the device is opened |
| 3 | Inhale | Flap 60 moves, dislodging prop 58; actuator 32 is triggered and cavity lid / separating element 20 is lifted |
| | | Actuator 32 pulls indexer link (pulling arm) 90 and the pawl 86 of the indexer / drive member 54 is ratcheted around the teeth 82 on the cavity disc. Indexer spring 52 is still relaxed. |
| 4 | Start to close device | Actuator32 is re-set against force of coil spring 46 by cam 44 on upper outer casing moving relative to actuator, closing lid (separating element 20) on emptied cavity. |
| | | Indexer spring 52 is re-set to energised state by force transmitting member 50 on upper outer casing 11. Brake still applied; disc does not move |
| | | Prop preventer 84 engages flap 60 and stops actuator32 from latching (to prevent possibility of latching the mechanism before indexing). |
| 5 | Finish closing device | Actuator32 remains in energised state - no change |
| | | Mouthpiece is enclosed by cover and external air inlet is blocked by housing. |
| | | Brake is released, allowing disc to be advanced by the indexer under the influence of the indexer spring. Prop preventer disengages from flap 60 to allow prop 58 to be moved against flap 60 under influence of prop spring 77. Prop 58 is in position to take load of spring-biased actuator when cam 44 disengages on opening. |

It should be noted that in this application terms such as "upper", "lower", "above", "below" have been used for explanatory purposes to describe the internal relationship between elements of the inhaler, regardless of how the inhaler is oriented in the surrounding environment. For instance, in the exemplified embodiment in the drawings, the cavities 16 are regarded as being placed "below" the foil layer 18, while the separating elements 20 are regarded as being placed "above" the foil layer 18, regardless of how the inhaler 2 as a whole is held or turned by the user. Similarly, "horizontal" means a direction located in the plane of the foil layer 18 or any plane parallel to the plane of the foil layer 18, and "vertical" means any direction perpendicular to such planes. Thus, a vertical line may intersect the cavities 16, the foil layer 18 and the separating elements 20.

## Claims

1. An inhaler (2) comprising:
- a housing (6, 8) and a mouthpiece (10) mounted on the housing, the mouthpiece having an inhalation channel (103),
- a disc shaped assembly (4) comprising a plurality of compartments (16) containing medicament, the disc assembly being rotatably mounted to the housing to allow medicament-containing compartments to be brought sequentially into registry with the mouthpiece, the mouthpiece being located at or beyond the outer periphery of the disc assembly and being in communication with the disc assembly via an aperture (5a, 5b) in the housing, and
- an indexing mechanism (10, 52, 54, 85, 86, 89, 90) associated with the disc assembly,
- **characterised in that** the mouthpiece (10) is movably mounted with respect to the housing (6, 8) and the mouthpiece forms part of the indexing mechanism.

2. An inhaler (2) as claimed in claim 1 wherein the mouthpiece (10) is a brake, being movable between (i) a braking position in which it is engaged with the disc assembly (4) and the disc assembly is thereby prevented from rotating and (ii) a released position in which it is disengaged from the disc assembly.

3. An inhaler (2) as claimed in claim 2 wherein the mouthpiece (10) includes a spacing member (107) which extends towards the disc assembly (4) and is engaged with a notch (108) in the disc assembly when in the braking position.

4. An inhaler (2) as claimed in claim 3 wherein the mouthpiece (10) and an outer cover component (11, 12) of the inhaler have cooperating cam surfaces (109, 105) such that relative rotation of the housing and outer cover component moves the mouthpiece between the braking position and the released position.

5. An inhaler (2) as claimed in claim 4 wherein the said cam surface (109) of the mouthpiece (10) is associated with a spring (106) which, in the braking position, acts to bias the spacing member (107) against the disc assembly (4).

6. An inhaler (2) as claimed in any one of claims 2 to 5 wherein, in the braking position, the mouthpiece (10) and the disc assembly (4) define a bypass air channel (111).

7. An inhaler (2) as claimed in claim 6 wherein a dimension of the bypass air channel (111) is determined by the spacing member (107) in engagement with the disc assembly (4).

8. An inhaler (2) as claimed in any one of the preceding claims, wherein
- the inhalation channel (103) extends though the mouthpiece and has an inhalation channel inlet,
- the disc shaped assembly (4) further comprises a plurality of respective medicament outlets, the disc assembly being rotatably mounted to the housing to allow the medicament outlets to be brought sequentially into registry with the inlet of the mouthpiece inhalation channel, and
- the said inlet being spaced from a said medicament outlet with which the mouthpiece is in registry, such that a bypass air channel (111) is defined between the medicament outlet and the inhalation channel inlet.

9. An inhaler (2) as claimed in claim 8 wherein the mouthpiece (10) includes a spacing member (107) which contacts an edge of the disc assembly (4) to define a dimension of the bypass air channel (111).

10. An inhaler (2) as claimed in claim 9 wherein the mouthpiece (10) is movably mounted such that the spacing member (107) may be brought out of contact with the disc assembly (4) to allow rotation of the disc assembly with respect to the housing (6, 8).

11. An inhaler (2) as claimed in claim 10 wherein a flexible mouthpiece seal member (9) forms a seal between the mouthpiece (10) and housing (6, 8), the seal member being able to accommodate movement of the mouthpiece to bring the spacing member (107) into and out of contact with the disc assembly (4) without breaking the sealing contact between the mouthpiece and the housing.

12. An inhaler (2) as claimed in claim 8 or claim 9 wherein the bypass air channel (111) extends around at least 80%, preferably 100% of a periphery of the inhalation channel inlet.

13. An inhaler (2) as claimed in claim 6, 7 or 12, wherein, in use, a composite air flow is created in the channel (103), air entering from the bypass channel (111) flowing nearer to the walls of the inhalation channel (103) than air flow entering from a medicament compartment (16) of the disc assembly.

14. An inhaler (2) as claimed in any of claims 7 to 10, further comprising secondary bypass air channels (104) extending through the mouthpiece.

15. An inhaler (2) as claimed in any of claims 7 to 11, further comprising a breath triggering mechanism (60) for triggering opening of medicament cavities (16) on inhalation by a user, and wherein substantially all inhaled air flow acts on the said breath triggering mechanism.

16. An inhaler (2) as claimed in any preceding claim containing an active ingredient selected from mometasone, ipratropium bromide, tiotropium and salts thereof, salemeterol, fluticasone propionate, beclomethasone dipropionate, reproterol, clenbuterol, rofleponide and salts, nedocromil, sodium cromoglycate, flunisolide, budesonide, formoterol fumarate dihydrate, terbutaline, terbutaline sulphate, salbutamol base and sulphate, fenoterol, 3-[2-(4-Hydroxy-2-oxo-3H-1,3-benzothiazol-7-yl)ethylamino]-N-[2-[2-(4-methylphenyl)ethoxy]ethyl]propane-sulphonamide, hydrochloride, indacaterol, aclidinium bromide, *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide or a pharmaceutically acceptable salt thereof (e.g. dihydrobromide); *N*-Cyclohexyl-*N*³-[2-(3-fluorophenyl)ethyl]-*N*-(2-{[2-(4-hydroxy-2-oxo-2,3-dihydro-1,3-benzothiazol-7-yl)ethyl]amino}ethyl)-β-alaninamide or a pharmaceutically acceptable salt thereof (e.g. di-D-mandelate); a [2-(4-Chloro-benzyloxy)-ethyl]-[2-((R)-cyclohexyl-hydroxy-phenyl-methyl)-oxazol-5-ylmethyl]- dimethyl-ammonium salt (e.g. hemi-naphthalene-1,5-disulfonate); a (*R*)-1-[2-(4-Fluoro-phenyl)-ethyl]-3-((*S*)-2-phenyl-2-piperidin-1-yl-propionyloxy)-1-azonia-bicyclo[2.2.2]octane salt (e.g. bromide or toluenesulfonate); or a combination of any two or more thereof.

## Patentansprüche

1. Inhalator (2), umfassend:
- ein Gehäuse (6, 8) und ein Mundstück (10), das an dem Gehäuse befestigt ist, wobei das Mundstück einen Inhalierkanal (103) aufweist,
- eine scheibenförmige Anordnung (4), umfassend mehrere Kammern (16), die Medikament enthalten, wobei die Scheibenanordnung drehbar an dem Gehäuse befestigt ist, damit die medikamenthaltigen Kammern nacheinander in Ausrichtung mit dem Mundstück gebracht werden, wobei das Mundstück am oder über den äußeren Umfang der Scheibenanordnung hinaus angeordnet ist und mit der Scheibenanordnung über eine Öffnung (5a, 5b) in dem Gehäuse verbunden ist, und
- einen Indexiermechanismus (10, 52, 54, 85, 86, 89, 90), der mit der Scheibenanordnung verbunden ist,
- **dadurch gekennzeichnet, dass** das Mundstück (10) beweglich in Bezug auf das Gehäuse (6, 8) befestigt ist und das Mundstück Teil des Indexiermechanismus ist.

2. Inhalator (2) nach Anspruch 1, wobei das Mundstück (10) eine Bremse ist, die zwischen (i) einer Bremsposition, in der es mit der Scheibenanordnung (4) in Eingriff steht, wodurch verhindert wird, dass sich die Scheibenanordnung dreht, und (ii) einer Freigabeposition, in der es von der Scheibenanordnung außer Eingriff gebracht wird bewegt werden kann.

3. Inhalator (2) nach Anspruch 2, wobei das Mundstück (10) ein Abstandselement (107) aufweist, das sich zu der Scheibenanordnung (4) erstreckt und mit einer Kerbe (108) in der Scheibenanordnung in Eingriff steht, wenn sich diese in der Bremsposition befindet.

4. Inhalator (2) nach Anspruch 3, wobei das Mundstück (10) und eine äußere Abdeckungskomponente (11, 12) des Inhalators zusammenwirkende Nockenoberflächen (109, 105) aufweisen, so dass eine relative Drehung des Gehäuses und der äußeren Abdeckungsomponente das Mundstück zwischen der Bremsposition und der Freigabeposition bewegt.

5. Inhalator (2) nach Anspruch 4, wobei die Nockenoberfläche (109) des Mundstücks (10) mit einer Feder (106) verbunden ist, die in der Bremsposition zum Vorspannen des Abstandselements (107) gegen die Scheibenanordnung (4) wirkt.

6. Inhalator (2) nach einem der Ansprüche 2 bis 5, wobei in der Bremsposition das Mundstück (10) und die Scheibenanordnung (4) einen Umleitungsluftkanal (111) definieren.

7. Inhalator (2) nach Anspruch 6, wobei eine Abmessung des Umleitungsluftkanals (111) von dem Abstandselement (107), das mit der Scheibenanordnung (4) in Eingriff steht, bestimmt wird.

8. Inhalator (2) nach einem der vorherigen Ansprüche, wobei:
- sich der Inhalierkanal (103) durch das Mundstück erstreckt und einen Inhalierkanaleinlass aufweist,
- die scheibenförmige Anordnung (4) ferner mehrere entsprechende Medikamentauslässe aufweist, wobei die Scheibenanordnung drehbar an dem Gehäuse befestigt ist, damit die Medikamentauslässe nacheinander in Ausrichtung mit dem Einlass des Mundstück-Inhalierkanals gebracht werden, und
- der Einlass von einem Medikamentenauslass beabstandet ist, mit dem das Mundstück ausgerichtet ist, sodass ein Umleitungsluftkanal (111) zwischen dem Medikamentenauslass und dem Inhalierkanaleinlass definiert wird.

9. Inhalator (2) nach Anspruch 8, wobei das Mundstück (10) ein Abstandselement (107) aufweist, das mit einem Rand der Scheibenanordnung (4) in Kontakt tritt, um eine Abmessung des Umleitungsluftkanals (111) zu definieren.

10. Inhalator (2) nach Anspruch 9, wobei das Mundstück (10) beweglich befestigt ist, sodass das Abstandselement (107) außer Kontakt mit der Scheibenanordnung (4) gebracht werden kann, um eine Drehung der Scheibenanordnung in Bezug auf das Gehäuse (6, 8) zu ermöglichen.

11. Inhalator (2) nach Anspruch 10, wobei ein flexibles Mundstück-Dichtungselement (9) eine Abdichtung zwischen dem Mundstück (10) und dem Gehäuse (6, 8) bildet, wobei das Dichtungselement die Bewegung des Mundstücks unterbringen kann, um das Abstandselement (107) in und außer Kontakt mit der Scheibenanordnung (4) zu bringen, ohne dass der Dichtungskontakt zwischen dem Mundstück und dem Gehäuse unterbrochen wird.

12. Inhalator (2) nach Anspruch 8 oder Anspruch 9, wobei der Umleitungsluftkanal (111) sich entlang mindestens 80%, vorzugsweise 100%, eines Umfangs des Inhalierkanaleinlasses erstreckt.

13. Inhalator (2) nach Anspruch 6, 7 oder 12, wobei während der Verwendung eine Verbundluftströmung in dem Kanal (103) erzeugt wird, wobei Luft, die aus dem Umleitungskanal (111) eintritt, näher an den Wänden des Inhalierkanals (103) strömt als die Luftströmung, die aus einer Medikamentenkammer (16) der Scheibenanordnung eintritt.

14. Inhalator (2) nach einem der Ansprüche 7 bis 10, ferner mit umfassend sekundäre Umleitungsluftkanäle (104), die sich durch das Mundstück erstrecken.

15. Inhalator (2) nach einem der Ansprüche 7 bis 11, ferner umfassend einen Atmungsauslösemechanismus (60) zum Auslösen des Öffnens der Medikamentenhohlräume (16) bei Inhalieren durch einen Benutzer, wobei im Wesentlichen die gesamte inhalierte Luftströmung auf den Atmungsauslösemechanismus wirkt.

16. Inhalator (2) nach einem der vorhergehenden Ansprüche, enthaltend einen Wirkstoff, der ausgewählt ist aus Mometason, Ipratropiumbromid, Tiotropium und Salzen davon, Salemeterol, Fluticasonpropionat, Beclomethasondipropionat, Reproterol, Clenbuterol, Rofleponid und Salzen, Nedocromil, Natriumcromoglycat, Flunisolid, Budesonid, Formoterolfumaratdihydrat, Terbutalin, Terbutalinsulfat, Salbutamolbase- und - sulfat, Fenoterol, 3-[2- (4-Hydroxy-2-oxo-3H-1,3-benzothiazol-7-yl)ethylamino]-N-[2- [2-(4-methylphenyl)ethoxy]ethyl]propan-sulfonamid, Hydrochlorid, Indacaterol, Aclidiniumbromid, N-[2-(Diethylamino)ethyl]-N-(2-([2-(4-hydroxy-2-oxo-2,3-dihydro-1,3-benzothiazol-7-yl)ethyl]amino)ethyl)-3-[2-(1-naphthyl)ethoxy]propanamid oder ein pharmazeutisch verträgliches Salz davon (z. B. Dihydrobromid); N-Cyclohexyl-*N*³-[2-(3-fluorophenyl)ethyl]-*N*-(2-([2-(4-hydroxy-2-oxo-2,3-dihydro-1,3-benzothiazol-7-yl)ethyl]amino)ethyl)-β-alaninamid oder ein pharmazeutisch verträgliches Salz davon(z. B. Di-D-mandelat); ein [2-(4-Chlor-benzyloxy) -ethyl]-[2-((R)-cyclohexyl-hydroxy-phenyl-methyl)oxazol-5 -ylmethyl]-dimethyl-Ammoniumsalz (z. B. Hemi-naphthalin-1,5-disulfonat), ein (*R*)-1-[2-(4-Fluoro-phenyl)-ethyl]-3-((*S*)-2-phenyl-2-piperidin-1-yl-propionyloxy)-1-azonia-bicyclo[2,2,2]octansalz (z. B. Bromid oder Toluolsulfonat) oder eine Kombination von beliebigen zwei oder mehr davon.

## Revendications

1. Inhalateur (2), comprenant:
- un boîtier (6, 8) et un embout buccal (10) monté sur le boîtier, l'embout buccal comprenant un canal d'inhalation 103),
- un ensemble en forme de disque (4) comprenant une pluralité de compartiments (16) contenant un médicament, l'ensemble de disque étant monté de façon rotative sur le boîtier de manière à permettre aux compartiments contenant un médicament d'être amenés de façon séquentielle en alignement avec l'embout buccal, l'embout buccal étant situé à ou au-delà de la périphérie extérieure de l'ensemble de disque et étant en communication avec l'ensemble de disque par l'intermédiaire d'une ouverture (5a, 5b) dans le boîtier, et
- un mécanisme d'indexation (10, 52, 54, 85, 86, 89, 90) associé à l'ensemble de disque,
**caractérisé en ce que** l'embout buccal (10) est monté de façon mobile par rapport au boîtier (6, 8), et que l'embout buccal fait partie du mécanisme d'indexation.

2. Inhalateur (2) selon la revendication 1, dans lequel l'embout buccal (10) est un frein, qui est mobile entre (i) une position de freinage, dans laquelle il est engagé avec l'ensemble de disque (4) et où l'ensemble de disque est de ce fait empêché de tourner, et (ii) une position libérée, dans laquelle il est désengagé de l'ensemble de disque.

3. Inhalateur (2) selon la revendication 2, dans lequel l'embout buccal (10) comprend un élément d'espacement (107) qui s'étend en direction de l'ensemble de disque (4) et qui est engagé avec une encoche (108) dans l'ensemble de disque lorsqu'il se trouve dans la position de freinage.

4. Inhalateur (2) selon la revendication 3, dans lequel l'embout buccal (10) et un composant de recouvrement extérieur (11, 12) de l'inhalateur présentent des surfaces de came coopérantes (109, 105) de telle sorte qu'une rotation relative du boîtier et du composant de recouvrement extérieur déplace l'embout buccal entre la position de freinage et la position libérée.

5. Inhalateur (2) selon la revendication 4, dans lequel ladite surface de came (109) de l'embout buccal (10) est associée à un ressort (106) qui, dans la position de freinage, agit pour pousser l'élément d'espacement (107) contre l'ensemble de disque (4).

6. Inhalateur (2) selon l'une quelconque des revendications 2 à 5, dans lequel, dans la position de freinage, l'embout buccal (10) et l'ensemble de disque (4) définissent un canal d'air de dérivation (111).

7. Inhalateur (2) selon la revendication 6, dans lequel une dimension du canal d'air de dérivation (111) est déterminée par l'élément d'espacement (107) en engagement avec l'ensemble de disque (4).

8. Inhalateur (2) selon l'une quelconque des revendications précédentes, dans lequel:
- le canal d'inhalation (103) s'étend à travers l'embout buccal et présente une entrée de canal d'inhalation,
- l'ensemble en forme de disque (4) comprend en outre une pluralité de sorties de médicament respectives, l'ensemble de disque étant monté de façon rotative sur le boîtier de manière à permettre aux sorties de médicament d'être amenées sensiblement en alignement avec l'entrée du canal d'inhalation de l'embout buccal, et
- ladite entrée étant espacée de ladite sortie de médicament avec laquelle l'embout buccal est aligné, de telle sorte qu'un canal d'air de dérivation (111) soit défini entre la sortie de médicament et l'entrée de canal d'inhalation.

9. Inhalateur (2) selon la revendication 8, dans lequel l'embout buccal (10) comprend un élément d'espacement (107) qui est en contact avec un bord de l'ensemble de disque (4) pour définir une dimension du canal d'air de dérivation (111).

10. Inhalateur (2) selon la revendication 9, dans lequel l'embout buccal (10) est monté de façon mobile de telle sorte que l'élément d'espacement (107) puisse être libéré de son contact avec l'ensemble de disque (4) afin de permettre la rotation de l'ensemble de disque par rapport au boîtier (6, 8).

11. Inhalateur (2) selon la revendication 10, dans lequel un élément de joint d'embout buccal flexible (9) forme un joint entre l'embout buccal (10) et le boîtier (6, 8), l'élément de joint étant capable de supporter le déplacement de l'embout buccal pour amener l'élément d'espacement (107) en contact et hors de contact avec l'ensemble de disque (4) sans rompre le contact étanche entre l'embout buccal et le boîtier.

12. Inhalateur (2) selon la revendication 8 ou la revendication 9, dans lequel le canal d'air de dérivation (111) s'étend sur environ au moins 80 %, de préférence 100 % d'une périphérie de l'entrée du canal d'inhalation.

13. Inhalateur (2) selon la revendication 6, 7 ou 12, dans lequel, lors de l'utilisation, un écoulement d'air composite est créé dans le canal (103), l'air entrant à partir du canal de dérivation (111) s'écoulant plus près des parois du canal d'inhalation (103) que le flux d'air entrant à partir d'un compartiment de médicament (16) de l'ensemble de disque.

14. Inhalateur (2) selon l'une quelconque des revendications 7 à 10, comprenant en outre des canaux d'air de dérivation secondaires (104) qui s'étendent à travers l'embout buccal.

15. Inhalateur (2) selon l'une quelconque des revendications 7 à 11, comprenant en outre un mécanisme de déclenchement de respiration (60) pour déclencher l'ouverture des cavités de médicament (16) lors de l'inhalation par un utilisateur, et dans lequel sensiblement la totalité du flux d'air inhalé agit sur ledit mécanisme de déclenchement de respiration.

16. Inhalateur (2) selon l'une quelconque des revendications précédentes, contenant un ingrédient actif sélectionné parmi la mométasone, le bromure d'ipratropium, le tiotropium et des sels de celui-ci, le salmétérol, le propionate de fluticasone, le dipropionate de béclométasone, le réprotérol, le clenbutérol, le rofléponide et ses sels, le nédocromile, le cromoglycate de sodium, le flunisolide, le budésonide, le dihydrate fumarate de formotérol, la terbutaline, le sulfate de terbutaline, le salbutamol base et sulfate, le fénotérol, le 3-[2-(4-hydroxy-2-oxo-3H-1,3-benzothiazol-7-yl)éthylamino]-N-[2-[2-(4-méthylphényle)éthoxy]éthyle]propane-sulfonamide, l'hydrochlorure, l'indacatérol, le bromure d'aclidinium, le N-[2-(diéthylamino)éthyle]-N-(2-{[2-(4-hydroxy-2-oxo-2,3-dihydro-1,3-benzothiazol-7-yle)éthyle]amino} éthyle)-3-[2-(1 - naphtyle)éthoxy]propanamide ou un sel de celui-ci acceptable sur le plan pharmaceutique (par exemple le dihydrobromure); le N-cyclohexyl-N³-[2-(3-fluorophényle)éthyle]-N-(2-{[2-(4-hydroxy-2-oxo-2,3-dihydro-1,3-benzothiazol-7-yle)éthyle]amino}éthyle)-β-alaninamide ou un sel de celui-ci acceptable sur le plan pharmaceutique (par exemple le di-D-mandélate); un sel de [2-(4-chloro-benzyloxy)-éthyle]-[2-((R)-cyclohexyle-hydroxy-phényle-méthyle)-oxazol-5-ylméthyle]- diméthyle-ammonium (par exemple l'hémi-naphtalène-1,5-disulfonate); un sel de (R)-1-[2-(4-fluoro-phényle)-éthyle]-3-((S)-2-phényle-2-pipéridine-l-yle-propionyloxy)-l-azonia-bicyclo[2.2.2]octane (par exemple le bromure ou le sulfonate de toluène);ou une combinaison de deux quelconques, ou plus, de ceux-ci.
